# EUROPÄISCHE PATENTSCHRIFT

(11) **EP 1 423 061 B1**
(45) Veröffentlichungstag und Bekanntmachung des Hinweises auf die Patenterteilung: **27.04.2005**
(21) Anmeldenummer: 02797666.1
(22) Anmeldetag: 03.09.2002
(51) Int. Cl.: A61F 5/01

(54) **KNIEGELENKORTHESE**
KNEE JOINT ORTHESIS
ORTHESE DE GENOU

(30) Priorität: 03.09.2001 DE 10143067
(43) Veröffentlichungstag der Anmeldung: 02.06.2004
(73) Patentinhaber: Bauerfeind AG, 07937 Zeulenroda (DE)
(72) Erfinder: BAUERFEIND, Hans, B., 07937 Zeulenroda (DE); REINHARDT, Holger, 47906 Kempen (DE); SCHEUERMANN, Rainer, 24223 Raisdorf (DE)
(74) Vertreter: Bardehle, Heinz, Dipl.-Ing.
(86) Internationale Anmeldenummer: PCT/EP2002/009841
(87) Internationale Veröffentlichungsnummer: WO 2003/020187

(56) Entgegenhaltungen:
- EP-A- 0 670 152
- US-A- 4 796 610
- US-A- 5 415 625
- US-A- 5 730 710

## Beschreibung

Die Erfindung betrifft eine Kniegelenkorthese mit oberhalb und unterhalb des Knies angeordneten, am Oberschenkel und Unterschenkel anlegbaren Haltern, die eine durch eine sich seitlich über das Knie erstreckende Gelenkschiene verbunden sind, die eine durch ein Fluid aufpumpbare Polsterung und im Bereich des Knies eine Gelenkverbindung aufweist.

Eine derartige Orthese ist in der europäischen Patentanmeldung 670 152 A1 offenbart. Bei der bekannten Kniegelenkorthese wird eine aufpumpbare Polsterung zur Korrektur der Beinlängsachse benutzt, die direkt seitlich neben dem Knie angeordnet ist und durch Aufpumpen sich so ausdehnt, dass sie dabei direkt auf das Knie einen Druck ausübt. Unter Berücksichtigung der Lage der um den Oberschenkel und den Unterschenkel gelegten kniefernen Halter wird dabei über die sich seitlich über das Knie erstreckende Gelenkschiene ein Moment auf das Knie ausgeübt, das dadurch je nach Lage der Gelenkschiene an der Beininnen- oder -aussenseite in Richtung einer X-Beinstellung oder O-Beinstellung gebracht werden soll. Dabei ergeben sich zwei Effekte: Einerseits wird direkt seitlich auf das Knie und damit auf die schmerzempfindliche Gelenkkapsel ein Druck ausgeübt, andererseits muss beim Beugen des Knies die aufpumpbare Polsterung auf der das Knie umgebenden Haut gleiten, was naturgemäß mit Reibung verbunden ist und bei längerem Tragen und insbesondere sportlicher Bewegung zum Wundscheuem der betreffenden Hautstellen führt.

Eine weitere Gestaltung einer Kniegelenkorthese zur Korrektur der Beinlängsachse ist durch die US-PS 5,302,169 bekannt geworden. Bei dieser Orthese werden die beiden sich über Oberschenkel und Unterschenkel erstreckenden Arme einer Gelenkschiene mittels Stellschrauben in eine jeweils gewünschte Schrägstellung zu einer Gelenkverbindung gebracht, die in relativ komplizierter Weise aus zwei miteinander verbundenen kugelgelenkartigen Gleitlagern besteht. Bei dieser Kniegelenkorthese sind auf das Knie wirkende Druckkräfte unvermeidlich, was das Tragen dieser Orthese, insbesondere bei Bewegung des Knies, erheblich beeinträchtigt. Darüber hinaus lässt die über relativ kurze Hebelarme erfolgende Schraubverstellung der beiden Arme der Gelenkschiene ein erhebliches Nachfedern dieser Arme zu, so dass mit der bekannten Kniegelenkorthese eine sichere therapeutisch erforderliche Korrektur der Beinstellung nicht gewährleistet werden kann.

Eine weitere Kniegelenkorthese ist in der US-PS 4,796,610 offenbart, gemäß der am Ende der Arme einer Gelenkschiene Kissen angebracht sind, die am Oberschenkel bzw. Unterschenkel anliegen. Durch diese Kniegelenkorthese sollen quer zum Bein im Bereich des Kniegelenks auftretende Kräfte aufgefangen und damit Verletzungen vermieden werden, wie sie insbesondere bei sportlicher Betätigung immer wieder vorkommen. Die Kniegelenkorthese dient also dazu, insbesondere Sportverletzungen zu vermeiden, sie ist nicht dafür konzipiert, eine bewusste Korrektur der Beinlängsachse herbeizuführen.

Es ist darüber hinaus z.B. aus der EP 0 684 026 A1 bekannt, an den Gelenkschienen einer Kniegelenkorthese zum Zwecke der Abpolsterung der Gelenkschienen gegen das Bein aufpumpbare Hohlkissen vorzusehen, die einzeln vom Träger so aufgeblasen werden können, dass ein möglichst unbeschwertes Tragen erzielt wird, d.h., die Hohlkissen dienen dazu, die Gelenkschienen individuell an die Gestaltung des betreffenden Beines, insbesondere auch an dessen Veränderung während des Tragens über einen Tag hin, anzupassen, um auf diese Weise eine besonders gute Passform zwischen der Kniegelenkorthese und dem Bein herbeizuführen.

Der Erfindung liegt die Aufgabe zugrunde, ausgehend von dem eingangs genannten Stand der Technik eine Kniegelenkorthese zu schaffen, die vom Patienten bequem individuell eingestellt werden kann und ein unbeschwerliches Tragen ermöglicht. Erfindungsgemäß geschieht dies gemäß einer ersten Version dadurch, dass die Polsterung zwei wahlweise aufpumpbare, durch einen Kanal verbundene Hohlkissen aufweist, die jeweils derart zwischen den Haltern und der Gelenkverbindung angeordnet sind, dass beim Aufpumpen der Hohlkissen das Knie gegenüber den Haltern in eine von der Gelenkverbindung entfernte Lage gedrückt wird.

Gemäß einer zweiten Version der Erfindung geschieht dies dadurch, dass die Polsterung zwei wahlweise aufpumpbare Hohlkissen aufweist, die jeweils derart neben den knienahen Haltern und entfernt von der Gelenkverbindung angeordnet sind, dass beim Aufpumpen der Hohlkissen Oberschenkel und Unterschenkel von der Gelenkschiene weggedrückt werden.

Durch die zwei wahlweise aufpumpbaren Hohlkissen lässt sich je nach dem Grad des Aufpumpens der Hohlkissen ein Moment erzeugen, durch das sowohl der Oberschenkel als auch der Unterschenkel in Bezug auf das Kniegelenk in einen jeweils gewünschten Winkel zu der Beinlängsachse eingestellt werden können. Die dabei auftretenden Drücke werden von den betreffenden Körperteilen ohne weiteres aufgefangen, da nämlich am Oberschenkel und Unterschenkel Bereiche existieren, die anatomisch geeignet sind, derartige Kräfte aufzunehmen. Auf jeden Fall bleibt das Knie selbst weitgehend von Druckkräften frei, so dass am Knie durch die Orthese selbst keine Schmerzen auftreten können und dieses auch nicht irgendwelchen Scheuereffekten ausgesetzt wird. Dabei gestatten die aufpumpbaren Hohlkissen einen von den betreffenden Patienten selbst einstellbaren Druck, der über an den Hohlkissen angebrachte Mundstücke und einen Pumpenschlauch mit daran angebrachter Handpumpe nach Art eines Pumpenballs ohne weiteres selbst die Hohlkissen aufpumpen kann, wobei ihm seine Schmerzlinderung hilft, den jeweils für ihn günstigsten Druck in den Hohlkissen stufenlos einzustellen. Auf Grund der Verbindung der beiden Hohlkissen durch einen Kanal stellt sich in beiden Hohlkissen immer der gleiche Druck ein, auch wenn nur eines der beiden Hohlkissen mit einem Ventil versehen ist, da sich auch in diesem Falle ein Druckausgleich über dem Kanal ergibt. Als Fluid kann eine Flüssigkeit, insbesondere aber auch ein Gas, wie Luft, verwendet werden.

Zur Stabilisierung der Gelenkverbindung lässt sich weiterhin ein sich über die Wadenseite erstreckender Gurt vorsehen, der an der der Gelenkverbindung abgewandten Seite der Kniegelenkorthese ansetzt und mit seinem freien Ende unterhalb der Gelenkverbindung festlegbar ist. Dieser Gurt bewirkt, dass eine beim Beugen des Knies auf die Gelenkverbindung wirkende Kraft, die diese vom Knie weg ziehen will, weitgehend kompensiert wird, so dass die Gelenkverbindung auch beim Beugen des Knies an ihrer notwendigerweise vorgesehenen Stellung verbleiben muss.

Das Aufpumpen der Hohlkissen wird dadurch erleichtert, dass man diese mit einem Ventil zum Ansetzen einer Pumpe versieht. Bei einer derartigen Pumpe kann es sich um eine bekannte Pumpe nach Art eines Pumpenballs handeln, wie er z.B. in Zusammenhang mit Blutdruckmessern verwendet wird.

Je nachdem, ob es sich darum handelt, eine mehr X-beinorientierte Korrektur oder mehr O-beinorientierte Korrektur herbeizuführen, wird die Gelenkschiene im Falle der ersten Version lateral (außen) zum Knie oder medial (innen) zum Knie angeordnet oder im Falle der zweiten Version medial oder lateral zum Knie.

Zweckmäßig gestaltet man die Kniegelenkorthese als Strumpf, in den dann die Halter und die Gelenkschiene eingebettet bzw. befestigt sind. Ein Strumpf lässt sich durch Überziehen über das Bein besonders leicht und bequem im Bereich des Knies formschlüssig anlegen, womit sich außerdem eine wünschenswerte Rutschfestigkeit ergibt.

Das Anlegen der Kniegelenkorthese lässt sich dadurch erleichtern, dass im Falle ihrer Ausbildung als Strumpf im Bereich der Kniescheibe zwei Profilpolster vorgesehen werden, die oberhalb und unterhalb der Kniescheibe diese derart einfassen, dass eine beim Beugen des Knies entstehende Spannung im Strumpf von der Kniescheibe weitgehend ferngehalten wird. Durch diese Profilpolster wird das Entstehen eines Druckes auf die Kniescheibe weitgehend verhindert, da die Spannung beim Beugen des Knies den Strumpf von Profilpolster zu Profilpolster spannt, ohne dass dabei die Kniescheibe mit beeinflusst werden kann. Im Übrigen dienen die Profilpolster dazu, beim Anlegen der Kniegelenkorthese diese von vornherein im Bezug auf die Kniescheibe an die richtige Stelle zu bringen, die durch den Patienten erfühlt wird, wenn die beiden Profilpolster sich oberhalb und unterhalb der Kniescheibe befinden.

Um den von den Hohlkissen auf den Oberschenkel und den Unterschenkel ausgehenden Druck zu vergleichsmäßigen, kann man vorteilhaft die Hohlkissen jeweils durch ein dem Oberschenkel bzw. Unterschenkel benachbartes Druckverteilungskissen abdecken. Ein solches Druckverteilungskissen kann beispielsweise aus weich eingestelltem Silikonkautschuk bestehen. Die Befestigung der Druckverteilungskissen gestaltet man dann zweckmäßig derart, dass diese von einer Tragplatte getragen werden, die einseitig an der Gelenkschiene verschwenkbar befestigt ist. Mit der Einstellung der Hohlkissen durch Einbringen des entsprechenden Druckes in diese kann sich dann die Tragplatte mit dem Druckverteilungskissen jeweils leicht schräg einstellen und sich damit der gewünschten Schrägstellung des Oberschenkels zum Unterschenkel anpassen.

Für die Befestigung der Hohlkissen eignet sich besonders die Gelenkschiene, an der dann die Hohlkissen dem Oberschenkel bzw. Unterschenkel benachbart befestigt sind, beispielsweise durch Festkleben. Es ist aber auch möglich, die Hohlkissen jeweils an einer Tragplatte zu befestigen.

Um den Hohlkissen ein zweckmäßiges Widerlager zu geben, sieht man zweckmäßig Stützplatten vor, die die Hohlkissen abdecken und jeweils an einer Gelenkschiene befestigt sind.

In den Figuren sind Ausführungsbeispiele der Erfindung dargestellt. Es zeigen:
- Figur 1: die Kniegelenkorthese gemäß der ersten Version in perspektivischer Sicht, und zwar angelegt lateral an einem rechten Bein;
- Figur 2: ist die gleiche Kniegelenkorthese in Seitensicht;
- Figur 3: ist die gleiche Kniegelenkorthese im Schnitt gemäß III-III aus Figur 2;
- Figur 4: die Gelenkschiene des Ausführungsbeispiels gemäß den Figuren 1 bis 3 in einer Sicht auf die dem Bein zugewandten Seite;
- Figur 5: die gleiche Kniegelenkorthese in einer Sicht auf die Kniekehle;
- Figur 6: eine Kniegelenkorthese gemäß der zweiten Version im Schnitt.

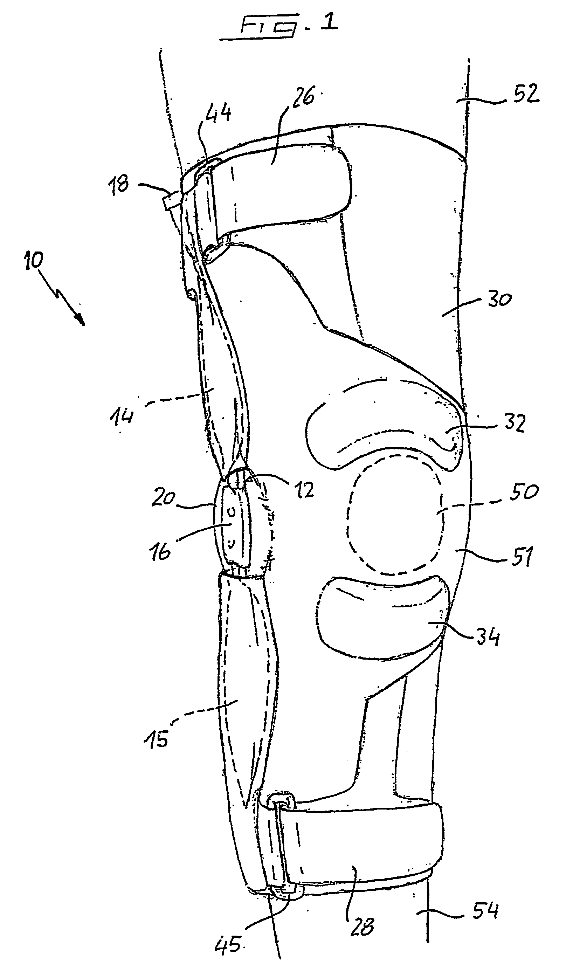

In der Figur 1 ist eine Kniegelenkorthese 10 der ersten Version, angelegt an den Oberschenkel 52 und den Unterschenkel 54, dargestellt, die den Strumpf 30 enthält, der über das Knie 51 gezogen ist, wobei die Lage des Knies 51 durch die gestrichelt eingezeichnete Kniescheibe 50 erläutert wird. Die Kniegelenkorthese 10 wird um den Oberschenkel 52 durch den Halter 26 und um den Unterschenkel 54 durch den Halter 28 gespannt. Damit ist die Kniegelenkorthese 10 in Bezug auf ihre Lage zum Oberschenkel 52 und Unterschenkel 54 weitgehend festgelegt.

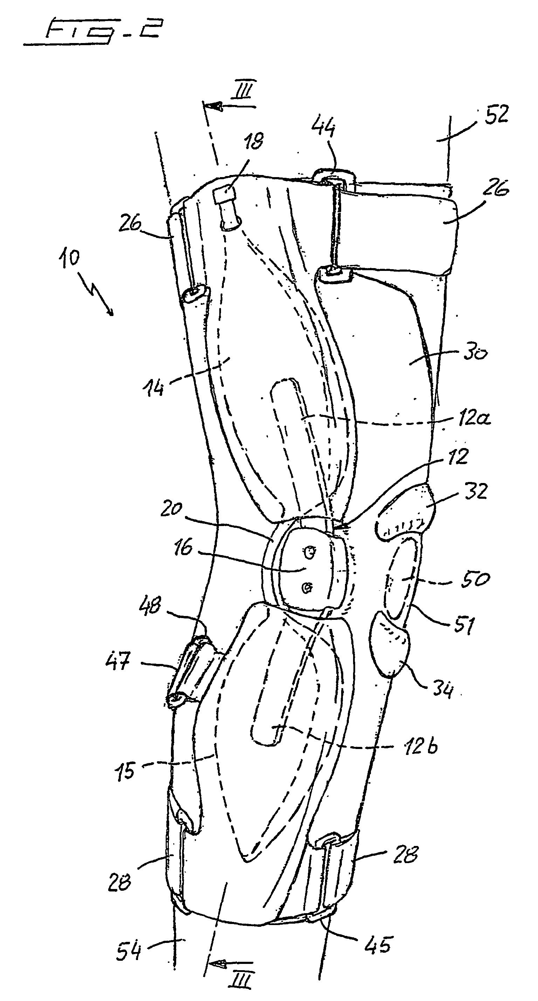

Die vorstehend benannten Teile der Kniegelenkorthese 10 sind ebenfalls in der Figur 2 dargestellt, die die Kniegelenkorthese in Seitensicht zeigt, und zwar auf die Lateralseite eines rechten Knies. In die Kniegelenkorthese 10 ist die Gelenkschiene 12a/12b eingearbeitet, die mit ihrem Arm 12a über einen Teil des Oberschenkels 52 und mit ihrem Arm 12b über einen Teil des Unterschenkels 54 verläuft und im Bereich des Knies 51 die Gelenkverbindung 16 aufweist. Aufgrund der Gelenkverbindung 16 kann die Gelenkschiene 12a/12b eine dem Oberschenkel 52 und Unterschenkel 54 folgende Beugung mitmachen.

Die Gelenkschiene 12a/12b weist eine aufpumpbare Polsterung; bestehend aus dem Hohlkissen 14 und dem Hohlkissen 15 auf, die jeweils zwischen den kniefernen Haltern 26 und 28 und der Gelenkverbindung 16 angeordnet sind. Zum Aufpumpen der Hohlkissen 14 und 15 dient das Ventil 18, über das in bekannter Weise Luft in die Hohlkissen 14 und 15 eingeblasen werden kann, worauf weiter unten im Einzelnen eingegangen wird. Hierzu wird an das Ventil 18 eine Pumpe angeschlossen, beispielsweise ein Pumpenball eingesteckt, wie er im Zusammenhang mit Blutdruckmessern verwendet wird.

Mit dem Aufblasen der Hohlkissen 14 und 15 ergibt sich ein Druck auf denjenigen Bereich des Oberschenkels 52 bzw. Unterschenkels 54, der zwischen den Haltern 26 bzw. 28 und der Gelenkverbindung 16 liegt, wodurch das Knie 51 von der Gelenkverbindung 16 in Richtung auf das andere Knie hin, also in Richtung X-Beinstellung, gedrückt wird, da die Gelenkschiene 12a/12b gegenüber den dabei auf sie wirkenden Kräften weitgehend starr bleibt.

Aufgrund dieses Wegdrückens des Knies 51 von der Gelenkverbindung 16 entsteht also der gewünschte therapeutische Effekt, in dem betreffenden Bein des Patienten das Knie 51 in eine mehr dem X-Bein angenäherte Lage zu bringen.

Es sei noch darauf hingewiesen, dass die Festlegung der Halter 26 und 28 in bekannter Weise durch Umschlingen des Oberschenkels 52 bzw. Unterschenkels 54 erfolgt, wobei dann das jeweilige Ende des Halters 26, 28 durch die Schnalle 44 bzw. 45 gezogen und danach durch Umlegen in bekannter Weise mittels Klettverschluss an dem umschlingenden Teil des Halters 26 bzw. 28 festgelegt wird.

Im Bereich des Knies 51 ist der Strumpf 30 mit den beiden Profilpolstern 32 und 34 versehen, die oberhalb und unterhalb der gestrichelt angedeuteten Kniescheibe 50 diese umfassen. Bei den Profilpolstern 32 und 34 kann es sich z.B. um Silikonpolster handeln, die in den Strumpf 30 eingesetzt sind. Durch die Profilpolster wird einerseits das Anlegen der Kniegelenkorthese insofern erleichtert, als der Patient fühlt, wenn der Bereich seiner Kniescheibe zwischen die Profilpolster 32 und 34 gelangt. Außerdem bewirken die Profilpolster 32 und 34, dass sich beim Beugen des Knies 51 das Material des Strumpfes 30 im Bereich des Knies 51 nicht direkt über der Kniescheibe 50 spannen kann, weil nämlich die Profilpolster 32 und 34 das Material des Strumpfes weitgehend von dem Bereich der Kniescheibe 50 fern halten.

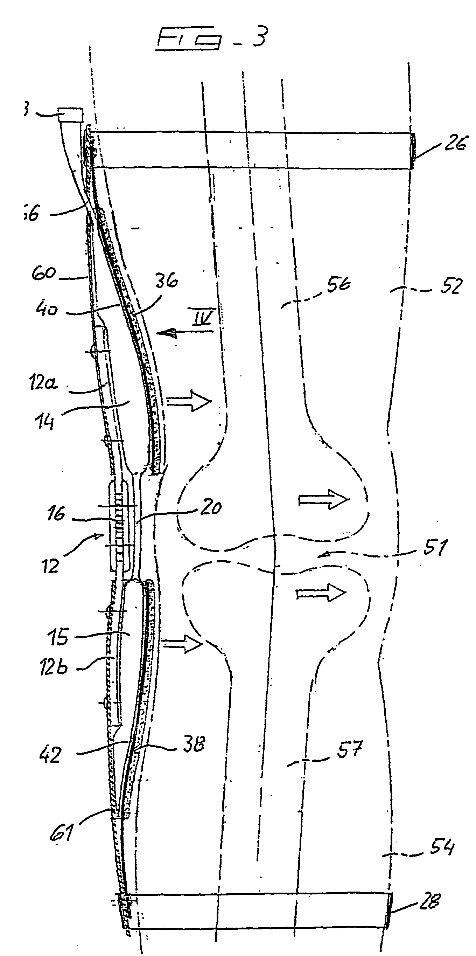

In der Figur 3 ist ein Längsschnitt durch die Kniegelenkorthese 10 gemäß den Figuren 1 und 2 (siehe Schnittlinie III-III in Figur 2) dargestellt. Dabei sind andeutungsweise das Knie 51, der Oberschenkelknochen 56 und der Unterschenkelknochen 57 sowie die Halter 26 und 28 dargestellt, wobei zur Vereinfachung der Darstellung die Schnallen 44 und 45 weggelassen sind. Außerdem ist auch der in den Figuren 1 und 2 mit 30 bezeichnete Strumpf nicht eingezeichnet, um die Gestaltung der Gelenkschiene 12a/12b mit den zu ihr gehörenden Bauteilen deutlich werden zu lassen.

Ausgehend von der Gelenkverbindung 16 ist die Gelenkschiene 12a/12b dargestellt, die sich von der Gelenkverbindung 16 sowohl in Richtung Oberschenkel 52 als auch Unterschenkel 54 erstreckt. Auf die Gelenkschiene 12a/12b ist die obere Stützplatte 60 und die untere Stützplatte 61 aufgenietet, die jeweils ein Hohlkissen abdecken, und zwar im Bereich des Oberschenkels 52 das Hohlkissen 14 und im Bereich des Unterschenkels 54 das Hohlkissen 15. Gemäß Figur 3 sind die Hohlkissen 14 und 15 in aufgeblasener Lage wiedergegeben, in der sie den knienahen Teil des Oberschenkels 52 und des Unterschenkels 54 von der Gelenkverbindung 16 wegdrücken und damit das betreffende Bein des Patienten mehr in die X-Beinlage drücken. Das Aufblasen der beiden Hohlkissen 14 und 15 erfolgt über das Ventil 18 und dem in das Hohlkissen 14 einmündenden Schlauch 66 sowie den Kanal 20, der das Hohlkissen 14 mit dem Hohlkissen 15 verbindet Auf diese Weise werden über das Ventil 18 beide Hohlkissen 14 und 15 gleichzeitig aufgeblasen. Auf der einen Seite stützen sich die Hohlkissen 14 und 15 gegen die Stützplatten 60 und 61 ab, auf der anderen Seite drücken die Hohlkissen 14 und 15 gegen die Tragplatten 40 und 42 und über diese auf die Druckverteilungskissen 36 und 38, wodurch letztere dafür sorgen, dass sich gegenüber dem Oberschenkel 52 bzw. Unterschenkel 54 örtlich kein zu hoher Druck ergibt

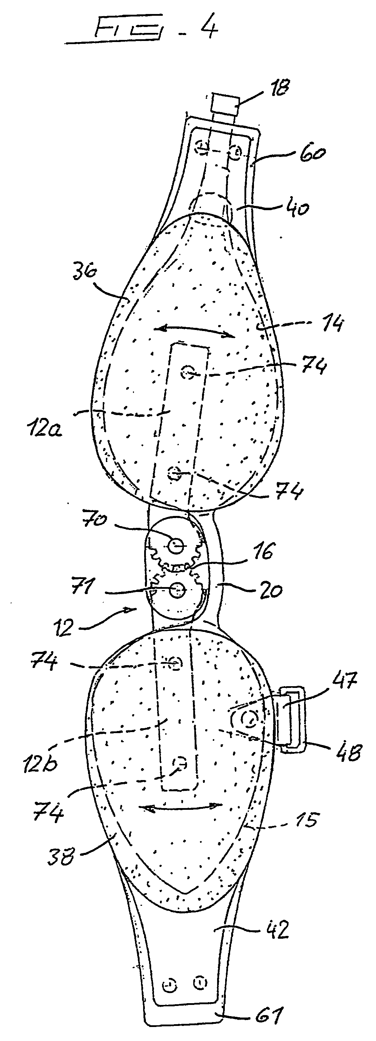

In der Figur 4 sind die mit der Gelenkschiene 12a/12b direkt in Verbindung stehenden Bauteile für sich allein dargestellt, um deren Funktion besonders verdeutlichen zu können. Gezeigt ist die Gelenkverbindung 16, die die beiden Achsen 70, 71 enthält, auf denen die beiden Arme der Gelenkschiene 12a/12b sitzen. Diese beiden Arme enden in jeweils einer Verzahnung, wobei die beiden Verzahnungen der beiden Arme ineinander greifen, so dass sich beim Beugen des Knies und damit der beiden Arme der Gelenkschiene 12a/12b ein Abwälzen im Bereich der Verzahnungen ergibt. Diese Art der gegenseitig bedingten Bewegung der beiden Arme der Gelenkschiene 12a/12b über die beiden Achsen 70 und 71 mit den beiden Verzahnungen ist in bekannter Weise der Gelenkbewegung des menschlichen Knies weitgehend angepasst. Die beiden Arme 12a und 12b sind über die Nieten 74 mit den beiden Stützplatten 60 und 61 fest verbunden, die auf das in Bezug auf die zeichnerische Darstellung darüberliegende Hohlkissen 14, 15 diese vollständig abdecken, so dass sich die Hohlkissen 14 und 15 gut gegenüber den Stützplatten 60 und 61 abstützen können. Beim Aufblasen der Hohlkissen 14 und 15 drücken diese die Tragplatten 40 und 42 gewissermaßen vor sich her und drücken damit die Druckverteilungskissen 36 und 38 von den Stützplatten 60 und 61 weg, womit dann der in Zusammenhang mit Figur 3 dargestellte Druck auf den Oberschenkel 52 und den Unterschenkel 54 ausgeübt wird.

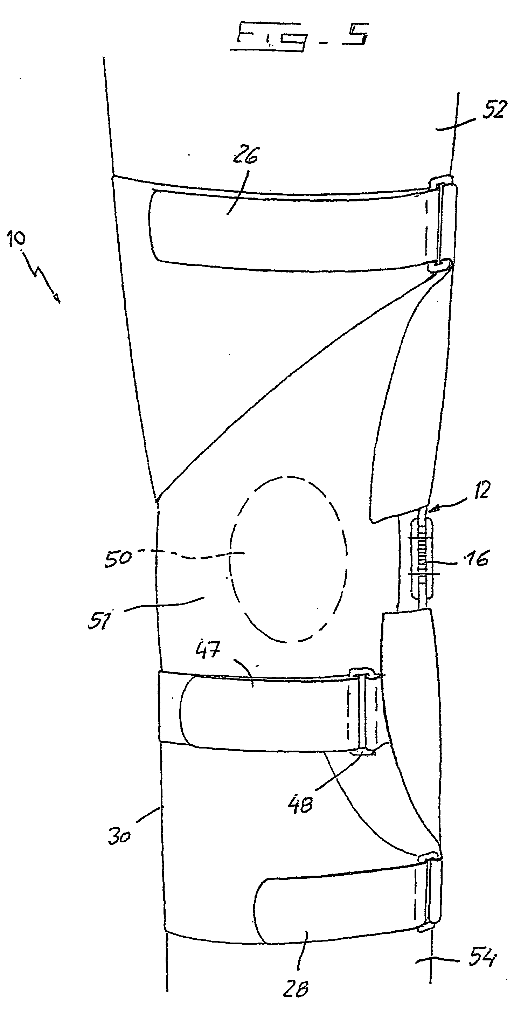

In den Figuren 2, 4 und 5 ist ein weiterer Bestandteil der erfindungsgemäßen Kniegelenkorthese dargestellt, der aus einem Gurt 47 besteht, der sich von der Seite der Kniegelenkorthese, die der Gelenkverbindung 16 abgewandt ist, also auf der gegenüberliegenden Seite der Kniegelenkorthese ansetzt, sich über die Wadenseite erstreckt und mit seinem freien Ende unterhalb der Gelenkverbindung 16 an dem Strumpf 30 festlegbar ist. Wie sich aus Figur 4 ergibt, ist der Gurt 47 mit der Schnalle 48 verbunden, die von dem Gurt 47 durchschlungen ist und im Bereich unterhalb der Kniekehle an dem Strumpf 30 festlegbar ist. Dies geschieht in bekannter Weise mittels eines Klettverschlusses. Durch diesen Gurt 47 wird über die Wadenseite von dem Bereich unterhalb der Kniekehle ein Zug auf die Gelenkverbindung 16 ausgeübt, wenn das Kniegelenk 51 gebeugt wird. In diesem Falle erhält nämlich die Gelenkverbindung 16 die Tendenz, in Richtung Kniescheibe 50 zu wandern, was durch den Gurt 47 verhindert wird. Durch diesen Gurt erhält also die Kniegelenkorthese eine besondere innere Stabilität.

Gemäß Darstellung in den vorstehend behandelten Figuren 1 bis 5 liegt, wie gesagt, die Gelenkschiene12a/12b auf der lateralen Seite eines rechten Kniegelenkes, womit bei Aufblasen der Hohlkissen 14 und 15 dem betreffenden Bein des Patienten eine in Richtung X-Bein tendierende Stellung aufgedrückt wird. Der gegenläufige Effekt bei der Streckung des Knies kann dadurch erzielt werden, dass die Kniegelenkorthese auf der medialen Seite des Beines eines Patienten angebracht wird, wodurch dann umgekehrt dem betreffenden Bein die Tendenz in Richtung O-Beinstellung gegeben wird.

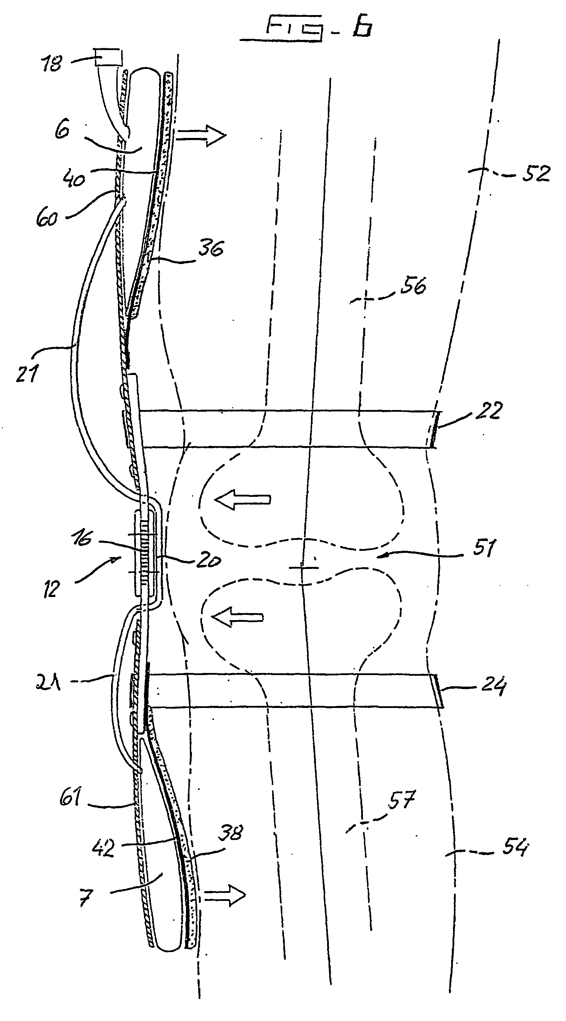

Eine weitere Möglichkeit der Erzeugung einer O-Beinstellung ist in Figur 6 dargestellt. Es handelt sich wieder um eine lateral angebrachte Kniegelenkorthese an einem rechten Bein. Bei der betreffenden Kniegelenkorthese liegen die Hohlkissen 6 und 7 an einer weiter vom Knie 51 entfernten Position, wobei zusätzlich die Halter 22 und 24 nahe dem Knie 51 vorgesehen sind. Die Hohlkissen 6 und 7 liegen also vom Knie 51 her gesehen außerhalb des Bereichs der Halter 22 und 24. Die Hohlkissen 6 und 7 sind wie bei der Kniegelenkorthese gemäß Figur 3 zwischen den Stützplatten 60 und 61 sowie den Tragplatten 40 und 42 angeordnet, so dass beim Aufblasen der Hohlkissen 6 und 7 über das Ventil 18 die Tragplatten 40 und 42 von den Stützplatten 60 und 61 weggedrückt werden. Dabei wird der Druck auf den Oberschenkel 52 und den Unterschenkel 54 über die Druckverteilungskissen 36 und 38 weitergeleitet. Insofern entspricht diese Funktion derjenigen, wie sie anhand der Figur 3 beschrieben ist.

Die Verbindung zwischen dem Hohlkissen 6 und dem Hohlkissen 7 erfolgt hier über den Kanal 21, durch den die in das Hohlkissen 6 eingepumpte Luft in das Hohlkissen 7 überströmen kann, so dass sich schließlich in beiden Hohlkissen der gleiche Druck einstellt. Der gegenläufige Effekt bei der Streckung des Knies kann dadurch erzielt werden, dass die Kniegelenkorthese auf der medialen Seite des Beines eines Patienten angebracht wird, wodurch dann umgekehrt dem betreffenden Bein die Tendenz in Richtung X-Beinstellung gegeben wird.

## Patentansprüche

1. Kniegelenkorthese (10) mit oberhalb und unterhalb des Knies angeordneten, am Oberschenkel (52) und Unterschenkel (54) anlegbaren Haltern (26,28), die durch eine sich seitlich über das Knie erstreckende Gelenkschiene verbunden sind, die eine durch ein Fluid aufpumpbare Polsterung und im Bereich des Knies eine Gelenkverbindung (16) aufweist, **dadurch gekennzeichnet, dass** die Polsterung zwei wahlweise aufpumpbare, durch einen Kanal verbundene Hohlkissen (14,15) aufweist, die jeweils derart zwischen den kniefernen Haltern (26,28) und der Gelenkverbindung (16) angeordnet sind, dass beim Aufpumpen der Hohlkissen (14,15) das Knie gegenüber den Haltern (26,28) in eine von der Gelenkverbindung (16) entfernte Lage gedrückt wird.

2. Kniegelenkorthese (10) mit oberhalb und unterhalb des Knies angeordneten, am Oberschenkel (52) und Unterschenkel (54) anlegbaren Haltern (26,28), die durch eine sich seitlich über das Knie erstreckende Gelenkschiene verbunden sind, die eine durch ein Fluid aufpumpbare Polsterung und im Bereich des Knies eine Gelenkverbindung (16) aufweist, **dadurch gekennzeichnet, dass** die Polsterung zwei wahlweise aufpumpbare Hohlkissen (14,15) aufweist, die jeweils derart neben den knienahen Haltern (26,28) und entfernt von der Gelenkverbindung (16) angeordnet sind, dass beim Aufpumpen der Hohlkissen (14,15) Oberschenkel (52) und Unterschenkel (54) von der Gelenkschiene weggedrückt werden.

3. Kniegelenkorthese nach Anspruch 1, **dadurch gekennzeichnet, dass** an ihrer der Gelenkverbindung abgewandten Seite ein über die Wadenseite sich erstreckender Gurt ansetzt, der mit seinem freien Ende unterhalb der Gelenkverbindung festlegbar ist.

4. Kniegelenkorthese nach einem der Ansprüche 1 bis 3, **dadurch gekennzeichnet, dass** mindestens ein Hohlkissen mit einem Ventil zum Ansetzen einer Pumpe versehen ist.

5. Kniegelenkorthese nach einem der Ansprüche 1 bis 4, **dadurch gekennzeichnet, dass** die Gelenkschiene lateral zum Knie angeordnet ist.

6. Kniegelenkorthese nach einem der Ansprüche 1 bis 4, **dadurch gekennzeichnet, dass** die Gelenkschiene medial zum Knie angeordnet ist.

7. Kniegelenkorthese nach einem der Ansprüche 1 bis 6, **dadurch gekennzeichnet, dass** die Halter und die Gelenkschiene in einem Strumpf eingebettet sind.

8. Kniegelenkorthese nach Anspruch 7, **dadurch gekennzeichnet, dass** der Strumpf im Bereich der Kniescheibe zwei Profilpolster aufweist, die oberhalb und unterhalb der Kniescheibe diese derart einfassen, dass eine beim Beugen des Knies entstehende Spannung im Strumpf von der Kniescheibe weitgehend ferngehalten wird.

9. Kniegelenkorthese nach einem der Ansprüche 1 bis 8, **dadurch gekennzeichnet, dass** die Hohlkissen jeweils durch ein dem Oberschenkel bzw. Unterschenkel benachbartes Druckverteilungskissen abgedeckt sind.

10. Kniegelenkorthese nach Anspruch 9, **dadurch gekennzeichnet, dass** das Druckverteilungskissen jeweils von einer Tragplatte getragen ist, die einseitig an der Gelenkschiene verschwenkbar befestigt ist.

11. Kniegelenkorthese nach einem der Ansprüche 1 bis 10, **dadurch gekennzeichnet, dass** die Hohlkissen an der Gelenkschiene befestigt sind.

12. Kniegelenkorthese nach Anspruch 10, **dadurch gekennzeichnet, dass** die Hohlkissen jeweils an der Tragplatte befestigt sind.

13. Kniegelenkorthese nach einem der Ansprüche 1 bis 12, **dadurch gekennzeichnet, dass** die Hohlkissen jeweils von einer an der Gelenkschiene befestigten Stützplatte abgedeckt sind.

## Claims

1. Knee-joint orthosis (10) with fasteners (26, 28) which are arranged above and below the knee, can be secured on the thigh (52) and lower leg (54) and are connected by a hinged bar extending laterally along the knee, said hinged bar having a padding that can be inflated by a fluid and, in the knee region, a hinged connection (16), **characterized in that** the padding has two selectively inflatable hollow cushions (14, 15) which are connected via a channel and are each arranged between the fasteners (26, 28), remote from the knee, and the hinged connection (16) in such a way that, upon inflation of the hollow cushions (14, 15), the knee is forced in relation to the fasteners (26, 28) into a position away from the hinged connection (16).

2. Knee-joint orthosis (10) with fasteners (26, 28) which are arranged above and below the knee, can be secured on the thigh (52) and lower leg (54) and are connected by a hinged bar extending laterally along the knee, said hinged bar having a padding that can be inflated by a fluid and, in the knee region, a hinged connection (16), **characterized in that** the padding has two selectively inflatable hollow cushions (14, 15) which are each arranged alongside the fasteners (26, 28) near to the knee and at a distance from the hinged connection (16) in such a way that, upon inflation of the hollow cushions (14, 15), the thigh (52) and the lower leg (54) are forced away from the hinged bar.

3. Knee-joint orthosis according to Claim 1, **characterized in that** a strap is attached to that side of the knee-joint orthosis facing away from the hinged connection, extends across the calf and can be fixed with its free end below the hinged connection.

4. Knee-joint orthosis according to one of Claims 1 to 3, **characterized in that** at least one hollow cushion is provided with a valve for attachment of a pump.

5. Knee-joint orthosis according to one of Claims 1 to 4, **characterized in that** the hinged bar is arranged laterally with respect to the knee.

6. Knee-joint orthosis according to one of Claims 1 to 4, **characterized in that** the hinged bar is arranged medially with respect to the knee.

7. Knee-joint orthosis according to one of Claims 1 to 6, **characterized in that** the fasteners and the hinged bar are embedded in a stocking.

8. Knee-joint orthosis according to Claim 7, **characterized in that**, in the region of the patella, the stocking has two contoured pads which are arranged above and below the patella and enclose the latter in such a way that a tension arising in the stocking when the knee is bent is kept well away from the patella.

9. Knee-joint orthosis according to one of Claims 1 to 8, **characterized in that** the hollow cushions are each covered by a pressure-distributing cushion adjoining the thigh and lower leg, respectively.

10. Knee-joint orthosis according to Claim 9, **characterized in that** the pressure-distributing cushion is in each case supported by a support plate which at one end is secured pivotably on the hinged bar.

11. Knee-joint orthosis according to one of Claims 1 to 10, **characterized in that** the hollow cushions are secured on the hinged bar.

12. Knee-joint orthosis according to Claim 10, **characterized in that** the hollow cushions are each secured on the support plate.

13. Knee-joint orthosis according to one of Claims 1 to 12, **characterized in that** the hollow cushions are each covered by a retaining plate secured on the hinged bar.

## Revendications

1. Orthèse de genou (10), avec des supports (26, 28) disposés au-dessus et au-dessous du genou et placés sur la cuisse (52) et la jambe (54), qui sont reliés par un rail articulé s'étendant latéralement le long du genou et possédant un rembourrage pouvant être gonflé d'un fluide et une liaison articulée (16) au niveau du genou, **caractérisée en ce que** le rembourrage présente deux coussins creux (14, 15) pouvant être gonflés sélectivement et reliés par un canal, qui sont disposés entre les supports (26, 28) et la liaison articulée (16) de telle manière que, lors du gonflage des coussins creux (14, 15), le genou est poussé par rapport aux supports (26, 28) dans une position éloignée de la liaison articulée (16).

2. Orthèse de genou (10), avec des supports (26, 28) disposés au-dessus et au-dessous du genou et placés sur la cuisse (52) et la jambe (54), qui sont reliés par un rail articulé s'étendant latéralement le long du genou et possédant un rembourrage pouvant être gonflé d'un fluide et une liaison articulée (16) au niveau du genou, **caractérisée en ce que** le rembourrage présente deux coussins creux (14, 15) pouvant être gonflés sélectivement et reliés par un canal, qui sont disposés entre les supports (26, 28) et la liaison articulée (16) de telle manière que, lors du gonflage des coussins creux (14, 15), la cuisse (52) et la jambe (54) sont éloignées de la liaison articulée (16).

3. Orthèse de genou selon la revendication 1, **caractérisée en ce que**, sur son côté opposé à la liaison articulée, se raccorde une courroie qui s'étend sur le côté du mollet et qui peut être fixée par son extrémité libre au-dessous de la liaison articulée.

4. Orthèse de genou selon l'une des revendications 1 à 3, **caractérisée en ce qu'**au moins un coussin creux est doté d'une valve pour le raccordement d'une pompe.

5. Orthèse de genou selon l'une des revendications 1 à 4, **caractérisée en ce que** le rail articulé est disposé latéralement par rapport au genou.

6. Orthèse de genou selon l'une des revendications 1 à 4, **caractérisée en ce que** le rail articulé est disposé médianement par rapport au genou.

7. Orthèse de genou selon l'une des revendications 1 à 6, **caractérisée en ce que** les supports et le rail articulé sont intégrés dans un bas.

8. Orthèse de genou selon la revendication 7, **caractérisée en ce que** le bas possède au niveau de la rotule deux rembourrements profilés placés au-dessus et au-dessous de la rotule et entourant celle-ci de telle manière que la rotule est largement protégée de la tension qui se produit dans le bas du fait de la flexion du genou.

9. Orthèse de genou selon l'une des revendications 1 à 8, **caractérisée en ce que** chacun des coussins creux est recouvert d'un coussin de distribution de la pression adjacent à la cuisse ou à la jambe.

10. Orthèse de genou selon la revendication 9, **caractérisée en ce que** le coussin de distribution de la pression est supporté par une plaque support qui est fixée unilatéralement sur le rail articulé de manière à pouvoir pivoter.

11. Orthèse de genou selon l'une des revendications 1 à 10, **caractérisée en ce que** les coussins creux sont fixés sur le rail articulé.

12. Orthèse de genou selon la revendication 10, **caractérisée en ce que** chacun des coussins creux est fixé sur la plaque support.

13. Orthèse de genou selon l'une des revendications 1 à 12, **caractérisée en ce que** chacun des coussins creux est recouvert par une plaque d'appui fixée sur le rail articulé.
